# EUROPEAN PATENT SPECIFICATION

(11) **EP 4 213 767 B1**
(45) Date of publication and mention of the grant of the patent: **06.05.2026**
(21) Application number: 20788924.7
(22) Date of filing: 21.09.2020
(51) Int. Cl.: A61F 2/16

(54) **INTRAOCULAR LENS**
INTRAOKULARLINSE
LENTILLE INTRAOCULAIRE

(43) Date of publication of application: 26.07.2023
(73) Proprietor: Carl Zeiss Meditec AG, 07745 Jena (DE)
(72) Inventor: SCHREIBER, Benjamin, 73447 Oberkochen (DE); BADUR, Thorben, 73447 Oberkochen (DE)
(74) Representative: PATERIS Patentanwälte PartmbB
(86) International application number: PCT/US2020/051837
(87) International publication number: WO 2022/060378

(56) References cited:
- WO-A1-2014/047345
- US-A1- 2016 206 421

## Description

The invention relates to an intraocular lens.

In cataract treatment of an eye, an incision is conventionally made in the cornea of the eye, said incision being large enough to allow a cannula to be inserted through the incision into the eye. After the incision has been made in the cornea, the lens of the eye is broken up by phacoemulsification and then sucked out of the capsular bag of the eye. Thereafter, an intraocular lens is inserted into the capsular bag by means of an injector. The intraocular lens includes an optical body and a haptic element, wherein the haptic element fixes the optical body in the capsular bag.

The haptic element has the function of keeping the optical body as close as possible to the middle of the eye in order to generate an image of maximum quality on the retina of the eye. Moreover, the optical body should be fixed with maximum positional stability in the capsular bag. In addition, the haptic element has the function of stopping the optical body from rotating about its optical axis. This is particularly relevant when the optical body is a toric optical body by means of which cornea curvature is to be corrected, because the toric optical body, if it is arranged in the capsular bag with an incorrect orientation, leads to an imaging aberration on the retina.

Once the intraocular lens has been inserted into the capsular bag, the intraocular lens may be disposed in an incorrect position in the capsular bag. This may be caused, for example, by nonuniform fibrosis. If the intraocular lens has been disposed in the wrong position in the capsular bag, it is necessary to correct the position of the intraocular lens in a surgical intervention or even to change the intraocular lens. Remedy could be provided by an intraocular lens having a correctable position after the insertion of the intraocular lens.

US 2016/0206421 A1 discloses an intraocular lens comprising a lens optic coupled to at least one haptic and at least one deformable connecting bar positioned between the lens optic and the at least one haptic. WO 2014/047345 A1 discloses intraocular lenses comprising at least one haptic having a shape memory alloy with a transition temperature substantially higher than the human body temperature, the shape memory alloy being post-surgically, selectively adjustable with a laser beam.

The problem addressed by the invention is therefore that of providing an intraocular lens having a correctable position after it has been inserted into a capsular bag of an eye.

The problem is solved by the features of claim 1. Preferred embodiments are specified in the dependent claims.

The intraocular lens of the invention has an optical body and a haptic element having a clip, a plastic bar that has a first longitudinal end secured to the optical body and a second longitudinal end to which the clip is secured and has a transition temperature higher than 40°C which is a glass transition temperature of the plastic bar or a melting temperature of the plastic bar, and at least one brace bar having a first longitudinal end secured to the optical body and a second longitudinal end to which the clip is secured.

If the intraocular lens has been inserted into the capsular bag of an eye, the clip is in contact with the capsular bag. The clip is in an extreme position relative to the optical body, the extreme position being the closest possible position of the clip to the optical body. The position of the optical body relative to the clip can be altered by heating the plastic bar and at least one of the at least one support bar by irradiating with electromagnetic radiation, especially by means of a laser. The plastic bar is heated here to temperatures above the transition temperatures, as a result of which the plastic bar becomes deformable and additionally lengthens. The heating of the at least one brace bar also causes it to lengthen. As a result, the clip moves out of the extreme position and away from the optical body. If irradiation of the plastic bar with the electromagnetic radiation is then ceased, the plastic bar cools down to temperatures below the transition temperature through conduction of heat. As a result, the plastic bar is no longer deformable, but instead firm, as a result of which the clip is outside the extreme position and is held there. In this way, it is possible to change the position of the optical body relative to the clip. If irradiation of the at least one brace bar with the electromagnetic radiation is then likewise ceased, this also cools down through conduction of heat. Because the second longitudinal end of the at least one brace bar is secured to the clip, this brace bar is now unable to shorten to the length that it had in the extreme position. This brace bar is under tensile stress once it has cooled down. By heating the plastic bar back to a temperature above the transition temperature and hence making it deformable, it is possible to move the clip back into the extreme position with loss of the tensile stress of the at least one brace bar. It is thus possible to reversibly change the position of the optical body relative to the clip.

The transition temperature is preferably from 40°C to 50°C, especially from 42°C to 50°C. These temperatures are only slightly above body temperature. As a result, the plastic bar need be heated only slightly for the plastic bar to become deformable. Moreover, no damage to body tissue is to be expected at these temperatures of the plastic bar. The transition temperature of the at least one brace bar, which is a glass transition temperature or a melting temperature of the brace bar, by contrast, is higher than 60°C, especially higher than 80°C. This can prevent the at least one brace bar from losing its tensile stress when the clip is disposed away from its extreme position.

The clip is preferably secured to the optical body solely via the plastic bar and the at least one brace bar. It is preferable that the at least one brace bar is arranged spaced apart from the plastic bar. More particularly, the at least one brace bar is arranged spaced apart from the plastic bar in circumferential direction with respect to the optical axis.

According to the invention, the at least one brace bar comprises a bimetal. It is preferable that the at least one brace bar comprises additionally a metal and/or a polymer or consists of the bimetal and optionally additionally of the metal and/or the polymer. The metal may, for example, comprise zinc or consist of zinc. The zinc may be encapsulated in an elastomer. Zinc advantageously has a high coefficient of thermal expansion. The bimetal may be formed, for example, by stainless steel and zinc. The polymer may comprise, for example, polypropylene and/or polyethylene or consist essentially of polypropylene and/or polyethylene. Polypropylene and polyethylene advantageously have a high coefficient of thermal expansion.

It is alternatively preferable that the at least one support bar comprises or consists of a substance having the structural formula

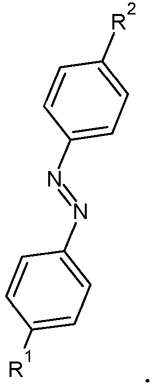

On irradiation with the electromagnetic radiation, the compound isomerizes from the E isomer shown above to the corresponding Z isomer. This is shown in the following reaction equation, where λ₁ symbolizes the irradiation:

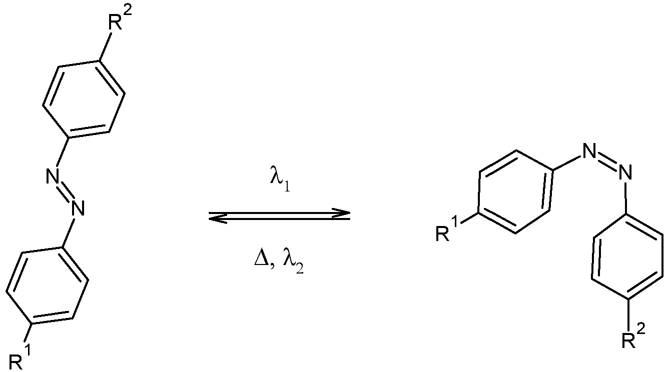

The Z isomer shown on the right has a lower density than the E isomer shown on the left, as a result of which the at least one brace bar expands on irradiation. If the irradiation ceases, the Z isomer can preferably isomerize back to the E isomer through body heat, represented symbolically in the reaction equation by Δ. Alternatively, irradiation with a wavelength λ₂, with λ₂ > λ₁, leads to conversion back to the E isomer. In this way, it is possible to regenerate the at least one brace bar.

It is preferable that R¹ is selected from the group of -H, -NR'R", -NH₂, -NHCOR', -OH, and -OR', where R' and R'' are independently an aliphatic radical, an aromatic radical, a polymerizable radical (for example acrylate, methacrylate or vinyl) or a polymeric radical (for example polyacrylate, polymethacrylate, polyvinyl), where R² is selected from the group of -H, -NR'R'', -NH₂, -NHCOR', -OH, -OR' and 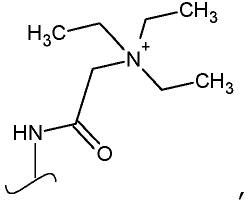 where R' and R'' are independently an aliphatic radical, an aromatic radical, a polymerizable radical (for example acrylate, methacrylate or vinyl) or a polymeric radical (for example polyacrylate, polymethacrylate, polyvinyl). An example of a conceivable counterion for the substance with the cation depicted above is a halide.

The at least one brace bar preferably has a coating having a low-pass filter for the electromagnetic radiation, where the low-pass filter is set up to allow the electromagnetic radiation having wavelengths longer than a limiting frequency to pass through to the substance with an intensity of less than 10% of an intensity of the electromagnetic radiation that hits the low-pass filter. More preferably, the low-pass filter is set up to allow the electromagnetic radiation having wavelengths longer than the limiting frequency to pass through to the substance with an intensity of less than 5% of an intensity of the electromagnetic radiation that hits the low-pass filter.

The plastic bar preferably comprises or consists of a copolymer of tert-butyl acrylate and poly(ethylene glycol) dimethacrylate. The copolymer is described in [1]. By means of the mass ratio of the two substances in the copolymer and the molecular mass of the copolymer, it is possible to adjust the glass transition temperature. The glass transition temperature can be measured, for example, by measuring its modulus of elasticity and/or its modulus of shear when heating the plastic bar. The glass transition temperature can be determined at the temperature at which the viscous state of the thermoresponsive polymer begins to form. One example of a plastic bar with a melting temperature is a block copolymer including diphenylmethane 4,4'-diisocyanate and butane-1,4-diol, as described in [2].

It is preferable that each of the brace bars has a conduit and the intraocular lens has a reservoir for each of the conduits that is connected to the respective conduit, wherein the reservoirs and the conduits contain a fluid. A change in the temperature of the fluid and/or the phase of the fluid changes the pressure within the reservoir and the conduit. By increasing the pressure in the conduit, it is possible to lengthen the brace bar. The provision of the reservoir results in a more significant rise in the pressure by comparison with lack of provision of the reservoir. Moreover, it is particularly easy for a laser to focus on the reservoir and hence to irradiate a large amount of the fluid. The change in the pressure is particularly high when the reservoir and the conduit are completely filled with the fluid. The phase is understood to mean a spatial region in which the material properties are homogeneous. A change in phase is understood to mean, for example, a transition from liquid to solid or from liquid to gaseous. A chemical transformation, for example from a dimer to a monomer, also constitutes a change in phase.

The at least one brace bar preferably comprises an elastomer. The elastomer may comprise a silicone and/or a polyacrylate or consist essentially of the silicone and/or the polyacrylate. Through the providing of the elastomer, it is easily possible for the brace bar to change length. It is particularly preferable that the elastomer bounds, especially fully bounds, the at least one conduit.

The at least one reservoir is preferably disposed in the region of the optical body or close to the optical body. The normal position of this region is not behind the iris of the eye but rather central in the capsular bag, and can therefore be reached easily by the electromagnetic radiation. Alternatively preferably, the at least one reservoir is arranged so as to adjoin the optical body behind the iris of the eye. Activation by means of electromagnetic radiation preferably follows prior medicament-induced broadening of the pupil.

It is preferable that the fluid contains a photosystem including an amount of a monomer and/or an amount of a dimer, wherein two of the monomers are set up to enter into a photoaddition with one another, especially a photocycloaddition, especially a [2+2] photocycloaddition, and hence to form the dimer, and the dimer is set up to enter into a photodissociation and hence to form two of the monomers. Such reactions are known to the person skilled in the art (see, for example, Jonathan Clayden, Nick Geeves, Stuart Warren, Organic Chemistry, Oxford University Press, 2006). For example, the photosystem for this purpose may include coumarin, a coumarin derivative, a cinnamic ester, especially methyl cinnamate, a cinnamic ester derivative, or a stilbene derivative, and/or the dimer of the aforementioned compounds. One example of photoaddition and photodissociation is depicted in reaction equation (1) below for coumarin and the coumarin derivative, with the dimer depicted on the right of the reaction arrow and the monomer on the left of the reaction arrow: 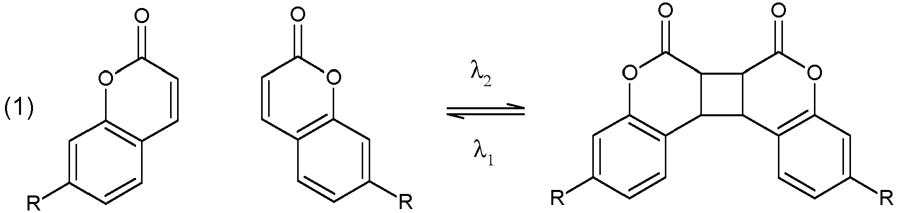 R may be, for example, -H, -NR'₂, -NH₂, -NHCOR', -OH or -OR', where the monomer is coumarin when R=-H. R' may be an aliphatic or aromatic radical or an acrylate. Irradiation with a second wavelength λ₂ leads to formation of the dimer; irradiation with a first wavelength λ₁ leads to dissociation of the dimer to the monomer. The dimer has a higher density than the monomer. Irradiation with the second wavelength λ₂ thus leads to lowering of the pressure in the conduit; irradiation with the first wavelength λ₁ leads to an increase in the pressure in the conduit.

A further example of photoaddition and photodissociation is depicted in reaction equation (2) below for methyl cinnamate, with the dimer depicted on the right of the reaction arrow and the monomer on the left of the reaction arrow:

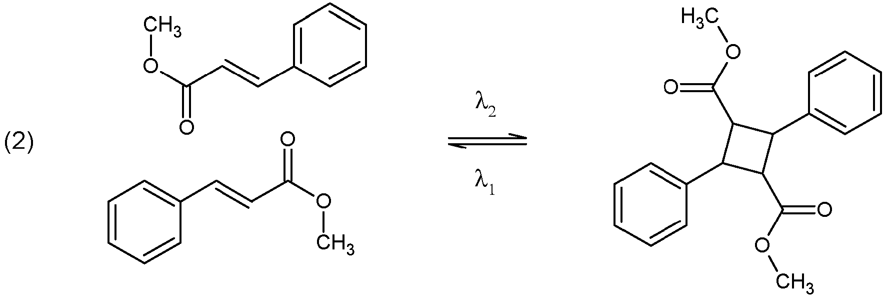

Irradiation with a second wavelength λ₂ leads to formation of the dimer; irradiation with a first wavelength λ₁ leads to dissociation of the dimer to the monomer. The dimer has a higher density than the monomer. Irradiation with the second wavelength λ₂ thus leads to lowering of the pressure in the conduit; irradiation with the first wavelength λ₁ leads to an increase in the pressure in the conduit.

A further example of photoaddition and photodissociation is depicted in reaction equation (3) below for a stilbene derivative, with the dimer depicted on the right of the reaction arrow and the monomer on the left of the reaction arrow:

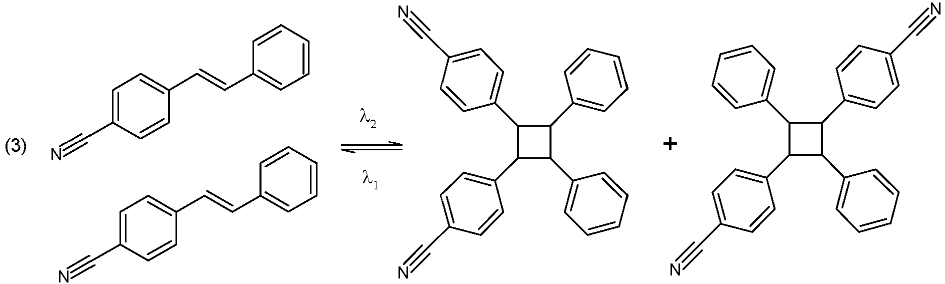

The dimer is present in two isomers. Irradiation with a second wavelength λ₂ leads to formation of the dimer; irradiation with a first wavelength λ₁ leads to dissociation of the dimer to the monomer. The dimer has a higher density than the monomer. Irradiation with the second wavelength λ₂ thus leads to lowering of the pressure in the conduit; irradiation with the first wavelength λ₁ leads to an increase in the pressure in the conduit.

It is preferable that the intraocular lens has a shutoff device for each of the brace bars that has a closed state in which flow of the fluid in a flow direction out of the reservoir belonging to the brace bar into the part of the conduit belonging to the brace bar arranged downstream of the flow direction and vice versa is prevented, or in which flow of the fluid in a flow direction out of the reservoir belonging to the brace bar into the part of the conduit belonging to the brace bar arranged downstream of the flow direction is enabled and in the reverse direction is prevented, and an open state in which the flow of the fluid in the flow direction into the part of the conduit belonging to the brace bar arranged downstream of the flow direction and vice versa is enabled. By means of the shutoff device, it is possible to move the shutoff device into the open state at a desired juncture and then to subject the fluid to the electromagnetic radiation in order to lengthen the brace bar. Moreover, by moving the shutoff device into the closed state, it is possible to prevent backflow of the fluid into the reservoir after the brace bar has been lengthened.

The shutoff device preferably comprises a bimetal set up to move the shutoff device from the closed state to the open state by means of an increase in temperature of the bimetal. The temperature of the bimetal can be increased by irradiating the bimetal with electromagnetic radiation, for example by means of a laser. As a result, it is not necessary to introduce a surgical instrument into the capsular bag in order to move the shutoff device into the open state. As a result of the bimetal cooling by conduction of heat, the shutoff device automatically returns to the closed state.

The shutoff device preferably has a photosystem containing an amount of a monomer and/or an amount of a dimer, wherein two of the monomers are set up to enter into a photoaddition with one another, especially a photocycloaddition, especially a [2+2] photocycloaddition, and hence to form the dimer and consequently move the shutoff device into its open state, and the dimer is set up to enter into a photodissociation and hence to form two of the monomers and consequently move the shutoff device into its closed state. The photosystem of the shutoff device may contain the same compounds as the photosystem of the fluid.

It is preferable that the intraocular lens includes a bandpass filter that has been disposed around the photosystem and is set up to allow a first wavelength range and a second wavelength range to pass through to the photosystem, wherein an irradiation of the photosystem in the first wavelength range leads to dissociation of the dimer and an irradiation of the photosystem in the second wavelength range leads to formation of the dimer. In this way, it is possible to prevent an unwanted phase transition of the photosystem resulting from incidence of daylight into the eye. The photosystem may, for example, be mixed with the bandpass filter and/or the bandpass filter may be disposed around the photosystem. It is particularly preferable that the first wavelength range includes the first wavelength λ₁ and the second wavelength range includes the second wavelength λ₂.

The haptic element preferably has two of the brace bars disposed in circumferential direction on either side of the plastic bar with respect to the optical axis of the optical body. By heating just one of the two brace bars or heating one of the two brace bars more strongly than the other of the two brace bars, it is possible to tilt the clip away from the extreme position. More preferably, the haptic element has fewer than three of the brace bars.

It is preferable that the at least one brace bar is arranged parallel to the plastic bar.

It is preferable that the intraocular lens has two of the haptic elements. More preferably, the two haptic elements are arranged symmetrically with respect to the optical axis of the optical body.

The invention is elucidated in detail with reference to the schematic drawings appended.
Figure 1 shows a top view of a preferred embodiment of the intraocular lens.
Figure 2 illustrates, using a first example, how the position of an optical body of the intraocular lens can be altered relative to a clip of the intraocular lens.
Figure 3 illustrates, using a second example, how the position of the optical body can be altered relative to the clip.
Figures 4 shows a top view of an alternatively preferred embodiment of the intraocular lens with a fluid-filled reservoir, connected conduit and shutoff device.
Figure 5 shows a first embodiment of a shutoff device of the intraocular lens.
Figure 6 shows a second embodiment of the shutoff device.
Figure 7 shows a third embodiment of the shutoff device.
Figure 8 shows a fourth embodiment of the shutoff device.
Figure 9 shows an illustrative bandpass filter of the intraocular lens.

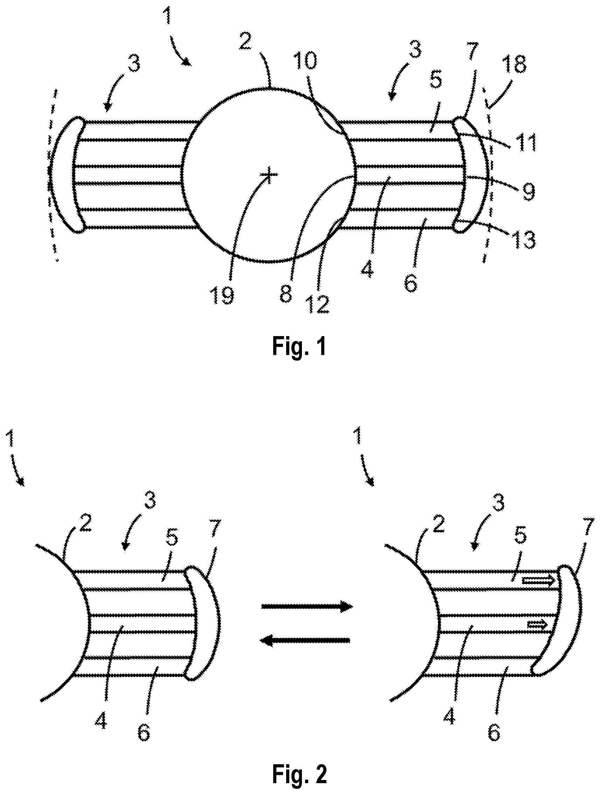

As apparent from figures 1 to 4, an intraocular lens 1 has an optical body 2 and a haptic element 3. The haptic element 3 has a clip 7, a plastic bar 4 and at least one brace bar 5, 6. The plastic bar 4 has a first longitudinal end 8 secured to the optical body 2, and a second longitudinal end 9 to which the clip 7 is secured. The plastic bar 4 has a transition temperature higher than 40°C, which is a glass transition temperature of the plastic bar 4 or a melting temperature of the plastic bar 4. The at least one brace bar 5, 6 has a first longitudinal end 10, 12 secured to the optical body 2, and a second longitudinal end 11, 13 to which the clip 7 is secured. The transition temperature of the plastic bar may, for example, be from 40°C to 50°C, especially from 42°C to 50°C. In addition, figures 1 to 4 show that the clip 7 is secured to the optical body 2 solely via the plastic bar 4 and the at least one brace bar 5, 6. It is additionally apparent that the clip 7 may have a side in convex form remote from the optical body 2. In addition, the clip 7 may have a side in concave form facing the optical body 2. Figure 1 shows that the intraocular lens 1 may have two of the haptic elements 3. One of the two haptic elements 3 may be arranged in a symmetric manner with respect to the other of the two haptic elements 3 with point symmetry at a point disposed on the optical axis 19 of the optical body 2.

Figures 1 to 4 show that the plastic bar 4 may have a longitudinal direction that may be arranged essentially at right angles to the optical axis 19 of the optical body 2. It is additionally apparent that the at least one brace bar 5, 6 may be arranged spaced apart from the plastic bar 4. It is additionally apparent that the at least one brace bar 5, 6 may have a longitudinal direction that may be arranged parallel to the longitudinal direction of the plastic bar 4. It is alternatively conceivable that the longitudinal direction of the at least one brace bar 5, 6 is arranged essentially at right angles to the optical axis 10 of the optical body 2.

It is conceivable that the haptic element 3 may have two of the brace bars 5, 6 that are disposed in circumferential direction on either side of the plastic bar 4 with respect to the optical axis 19 of the optical body 2, as also shown in figures 1 to 4.

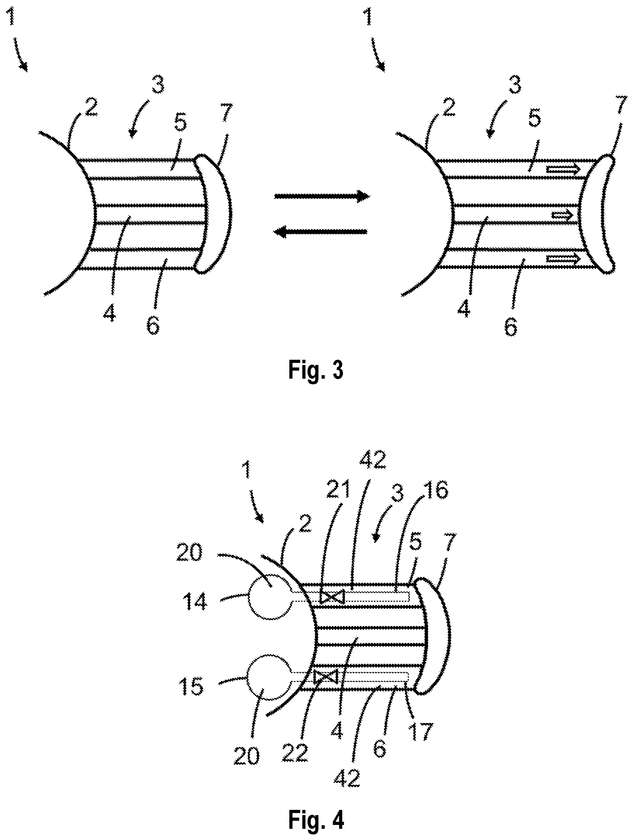

In a first example according to figure 2 and in a second example according to figure 3, it is shown how the position of the optical body 2 can be altered relative to the clip 7. In the examples, the haptic element 3 has a first brace bar 5 and a second brace bar 6 disposed in circumferential direction on either side of the plastic bar 4 with respect to the optical axis 19 of the optical body 2. In the left-hand drawings in figures 2 and 3, an extreme position of the clip 7 is shown, which is the closest possible position of the clip 7 to the optical body 2.

In the first example, the plastic bar 4 is heated to a temperature higher than the transition temperature, and the first brace bar 5 is heated, as a result of which the plastic bar 4 and the first brace bar 5 lengthen, which is illustrated by the arrows in the right-hand drawing in figure 2. The clip 7 moves from its extreme position and away from the optical body 2. Because the second brace bar 6 is not being heated, the clip 7 executes a tilting motion. Because the plastic bar 4 enables cooling to a temperature below the transition temperature, it is possible to freeze the position of the clip 7. It is conceivable here that the clip 7 will remain at least partly tilted. By heating the plastic bar 4 back to a temperature above the transition temperature, the clip 7 can be moved back to the extreme position.

In the second example, the plastic bar 4 is heated to a temperature above the transition temperature, and the first brace bar 5 and the second brace bar 6 are heated, as a result of which the plastic bar 4, the first brace bar 5 and the second brace bar 6 lengthen, which is illustrated by the arrows in the right-hand drawing in figure 3. In the second example, the brace bars 5, 6 are lengthened more than the plastic bar 4. This achieves the effect that the side remote from the optical body 2 that was convex in the extreme position becomes concave. An additional effect achieved is that the side facing the optical body 2 that was concave in the extreme position becomes convex. Because the plastic bar 4 enables cooling to a temperature below the transition temperature, it is possible to freeze the position of the clip 7. By heating the plastic bar 4 back to a temperature above the transition temperature, the clip 7 can be moved back to the extreme position.

It is conceivable that the at least one brace bar 5, 6 according to figures 1 to 3 comprises a metal, a bimetal and/or a polymer or consists of the metal, the bimetal and/or the polymer.

Figure 4 shows that each of the at least one brace bar 5, 6 may have a conduit 16, 17 and the intraocular lens 1 may have a reservoir 14, 15 for each of the conduits 16, 17 that is connected to the respective conduit 16, 17, wherein the reservoirs 14, 15 and the conduits 16, 17 contain a fluid 20. As apparent from figure 4, the reservoir 14, 15 may be broader than the overall conduit 16, 17 in a cross section having a normal parallel to the optical axis 19 of the optical body 2. The at least one brace bar 5, 6 may include an elastomer 42 that bounds and especially fully bounds the conduit 16, 17. As apparent from figure 4, the at least one reservoir 14, 15 may be disposed in the region of the optical body 2. It is alternatively conceivable that the at least one reservoir 14, 15 is disposed in a region adjoining the optical body 2.

Figure 4 shows that the intraocular lens 1 may have a shutoff device 21, 22 for each of the brace bars 5, 6 that has a closed state in which flow of the fluid 20 in a flow direction 24 out of the reservoir 14, 15 belonging to the brace bar 5, 6 into the part of the conduit 16, 17 belonging to the brace bar 5, 6 arranged downstream of the flow direction 24 and vice versa is prevented, or in which flow of the fluid 20 in a flow direction 24 out of the reservoir 14, 15 belonging to the brace bar 5, 6 into the part of the conduit 16, 17 belonging to the brace bar 5, 6 arranged downstream of the flow direction 24 is enabled and in the reverse direction is prevented, and an open state in which the flow of the fluid 20 in the flow direction 24 into the part of the conduit 16, 17 belonging to the brace bar 5, 6 arranged downstream of the flow direction 24 and vice versa is enabled. Figure 4 also shows that the shutoff device 21, 22 may be disposed in the conduit 16, 17 that belongs to the shutoff device 21, 22.

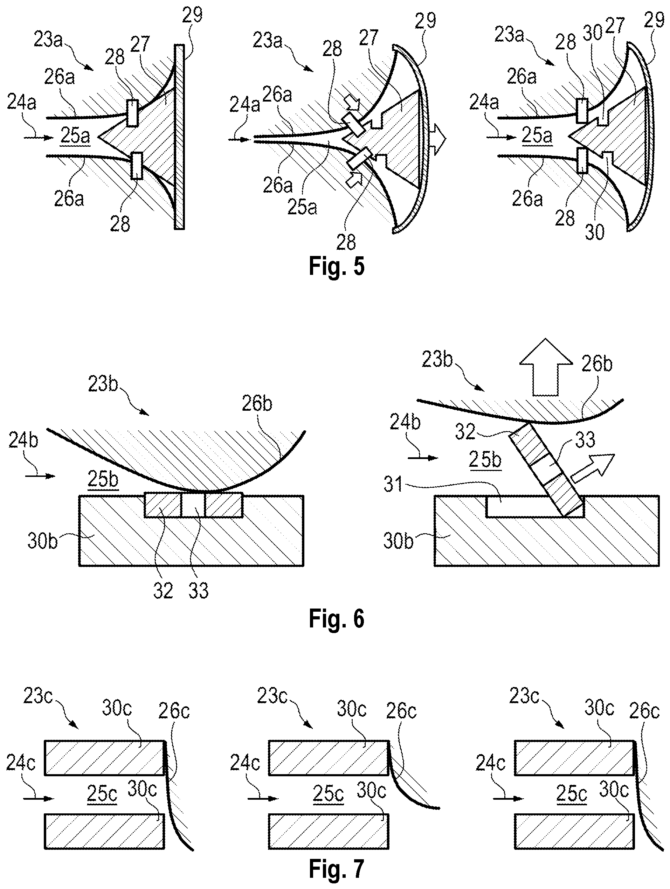

In the first embodiment of the shutoff device 23a according to figure 5, the shutoff device 23a has a shutoff device conduit 25a into which the fluid enters from the reservoir 14, 15 in the flow direction 24a, and the cross section of which widens in the flow direction 24a. The shutoff device conduit 25a is formed by the bimetal 26a. At the end face of the shutoff device conduit 25a, the shutoff device 23a has a plug 27 which is set up to block the shutoff device conduit 25a in the region where the shutoff device conduit 25 becomes wider, so that the fluid cannot exit from the shutoff device 23a. Figure 5 illustrates the shutoff device 23a at three different times, with time advancing from left to right. At the first time, the shutoff device 23a is blocked by the plug 27 and the shutoff device 23a is in its closed state; the shutoff device 23a is brought into the open state at the second time and the shutoff device 23a is in its open state at the third time. The shutoff device 23a has a bar 28, which is fastened to the bimetal 26a and which engages in a plug cutout 30 in the plug 27 in the closed state. In order to bring the shutoff device 23a into the open state, the bimetal 26a should be heated, as a result of which its curvature increases in the region of the shutoff device conduit 25a where the cross section increases, as a result of which the bar departs from the plug cutout 30, as illustrated for the second time. In the open state, the bar 28 keeps the plug apart from the bimetal 26a. The flow 24a pushes the plug away from the bar 28 at the third juncture and there is consequently flow between the bar 28 and the plug 27. It is conceivable for the shutoff device 23a to function like a valve because flow counter to 24a has the effect that the plug 27 is pressed onto the bar 28 and the fluid is consequently unable to pass the shutoff device counter to the flow direction 24a. The shutoff device 25a can have a holding part 29, which is configured to pretension the plug 27 in the direction counter to the flow direction 24a. By way of example, the holding part 29 could have one or more bands, with the fluid being able to pass the holding part 29 next to and/or between the bands.

According to the second embodiment of the shutoff device 23b as per figure 6, the shutoff device 23b has a shutoff device conduit 25b, into which the fluid enters from the reservoir 14, 15 in the flow direction 24b. In figure 6, the shutoff device 23b is illustrated in the closed state in the left-hand image and the open state in the right-hand image. The shutoff device 23b has a wall 30b delimiting the shutoff device conduit 25b, the wall having a wall cutout 31 in which a lever 32 is disposed in the closed state. At one longitudinal end of the lever 32, the lever 32 is attached to the wall 30b. The other longitudinal end of the lever 32 is pretensioned away from the wall by means of pretensioning means. The bimetal 26b delimits the shutoff device conduit 25b opposite to the wall cutout 31. In the closed state, the bimetal 26b presses the lever 32 into the wall cutout 31. In the open position, the bimetal 26b has a lower curvature than in the open position, and so the lever 32 is pressed out of the wall cutout 31 by the pretensioning means. The lever 32 can have a lever through-hole 33, through which the fluid can flow in the open state. In the second embodiment of the shutoff device 23b, the shutoff device 23b can be switched as desired between the open state and the closed state.

According to the third embodiment of the shutoff device 23c as per figure 7, the shutoff device 23c has a shutoff device conduit 25c into which the fluid enters from the reservoir 14, 15 in the flow direction 24c. The shutoff device conduit 25c is delimited by a wall 30c in the circumferential direction. The bimetal 26c is disposed at the end face of the shutoff device conduit 25c and is configured to open and close the end face by way of its curvature and consequently switch the shutoff device 23c between its open state and its closed state.

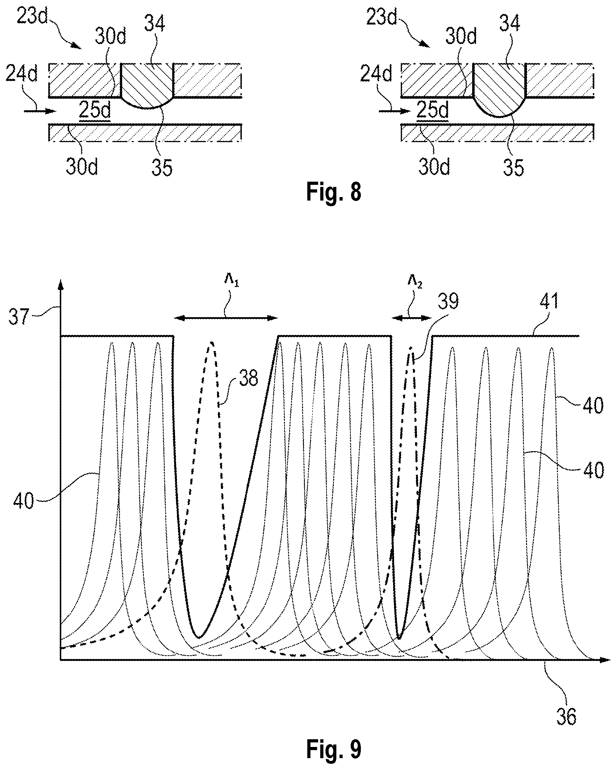

In the fourth embodiment of the shutoff device 23d according to figure 8, the shutoff device 23d has a photosystem 34 containing an amount of a monomer and/or an amount of a dimer, wherein two of the monomers are set up to enter into a photoaddition with one another, especially a photocycloaddition, especially a [2+2] photocycloaddition, and hence to form the dimer and consequently move the shutoff device 23d into its open state, and the dimer is set up to enter into a photodissociation and hence to form two of the monomers and consequently move the shutoff device 23d into its closed state. In the fourth embodiment of the shutoff device 23d according to figure 8, the shutoff device 23d has a shutoff device conduit 25d, into which the fluid enters from the reservoir 14, 15 in the flow direction 24d. The shutoff device 23d has a wall 30d, which delimits the shutoff device conduit 25d. The wall 30d has a cutout, in which the photosystem 34 is accommodated. A flexible wall 35 delimits the shutoff device conduit 25d in the region of the cutout. By reducing the density of the photosystem 34, the flexible wall 35 can be deformed to such an extent that it contacts the section of the wall 30d facing the cutout and consequently closes the shutoff device conduit 25d. As a result, the shutoff device 23d can be brought into its closed state and into its open state.

It is conceivable that the fluid 20 contains a photosystem including an amount of a monomer and/or an amount of a dimer, wherein two of the monomers are set up to enter into a photoaddition with one another, especially a photocycloaddition, especially a [2+2] photocycloaddition, and hence to form the dimer, and the dimer is set up to enter into a photodissociation and hence to form two of the monomers. For example, the photosystem of the fluid or of the shutoff device may comprise the coumarin, coumarin derivative, cinnamic ester (especially methyl cinnamate), cinnamic ester derivative, or stilbene derivative compounds already described, and/or the dimer of the aforementioned compounds.

It is conceivable for the intraocular lens 1 to have a bandpass filter that is disposed around the photosystem 34 and configured to allow a first wavelength range Λ₁ and a second wavelength range Λ₂ to pass through to the photosystem 34, wherein an irradiation of the photosystem 34 in the first wavelength range Λ₁ leads to dissociation of the dimer and an irradiation of the photosystem 34 in the second wavelength range Λ₂ leads to formation of the dimer.

Figure 9 shows an example of the absorption spectrum of the bandpass filter. The wavelength 36 is plotted across the abscissa, and the absorption 37 is plotted across the ordinate. The bandpass filter has a multitude of different absorbers that each have an individual absorption band 40. All the individual absorption bands 40 cumulatively result in an overall absorption 41 of the bandpass filter. The overall absorption 41 is smaller in the first wavelength range Λ₁ and in the second wavelength range Λ₂ than in the adjacent wavelength ranges. The absorption band 38 of the dimer is disposed in the first wavelength range Λ₁, and the absorption band 39 of the monomer is disposed in the second wavelength range Λ₂. Figure 9 shows that the first wavelength range Λ₁ and the second wavelength range Λ₂ are separated from one another. Alternatively, it is conceivable for the first wavelength range Λ₁ and the second wavelength range Λ₂ to overlap one another.

### Literature:

[1] T. Govindarajan, R. Shandas, Polymers 2017, 9(11), 572.
[2] B. Kim, S.Y. Lee, M Xu, Polymer 1996, 37(26), 5781-5793.

### List of reference numerals

1 Intraocular lens
2 Optical body
3 Haptic element
4 Plastic bar
5 First brace bar
6 Second brace bar
7 Clip
8 First longitudinal end of the plastic bar 4
9 Second longitudinal end of the plastic bar 4
10 First longitudinal end of the first brace bar 5
11 Second longitudinal end of the first brace bar 5
12 First longitudinal end of the second brace bar 6
13 Second longitudinal end of the second brace bar 6
14 First reservoir
15 Second reservoir
16 First conduit
17 Second conduit
18 Capsular bag
19 Optical axis
20 Fluid
21 First shutoff device
22 Second shutoff device
23a Shutoff device
23b Shutoff device
23c Shutoff device
23d Shutoff device
24a Flow direction
24b Flow direction
24c Flow direction
24d Flow direction
25a Shutoff device conduit
25b Shutoff device conduit
25c Shutoff device conduit
25d Shutoff device conduit
26a Bimetal
26b Bimetal
26c Bimetal
27 Plug
28 Bar
29 Holding part
30 Plug cutout
30b Wall
30c Wall
30d Wall
31 Wall cutout
32 Lever
33 Lever through-hole
34 Photosystem
35 Flexible wall
36 Wavelength
37 Absorption
38 Dimer absorption band
39 Monomer absorption band
40 Individual absorption band
41 Overall absorption
42 Elastomer
λ₁ First wavelength
λ₂ Second wavelength
Λ₁ First wavelength range
Λ₂ Second wavelength range

## Claims

1. An intraocular lens with an optical body (2) and a haptic element (3) having a clip (7), a plastic bar (4) that has a first longitudinal end (8) secured to the optical body (2) and a second longitudinal end (9) to which the clip (7) is secured and has a transition temperature higher than 40°C which is a glass transition temperature of the plastic bar (4) or a melting temperature of the plastic bar (4), and at least one brace bar (5, 6) having a first longitudinal end (10, 12) secured to the optical body (2) and a second longitudinal end (11, 13) to which the clip (7) is secured, **characterized in that** the at least one brace bar (5, 6) comprises a bimetal.

2. The intraocular lens as claimed in claim 1, wherein the clip (7) is secured to the optical body (2) solely via the plastic bar (4) and the at least one brace bar (5, 6).

3. The intraocular lens as claimed in claim 1 or 2, wherein the at least one brace bar (5, 6) is arranged spaced apart from the plastic bar (4).

4. The intraocular lens as claimed in any of claims 1 to 3, wherein the haptic element (3) has two of the brace bars (5, 6) disposed in circumferential direction on either side of the plastic bar (4) with respect to the optical axis (19) of the optical body (2).

5. The intraocular lens as claimed in any of claims 1 to 4, wherein the intraocular lens (1) has two of the haptic elements (3).

## Patentansprüche

1. Intraokularlinse mit einem optischen Körper (2) und einem haptischen Element (3), das eine Klammer (7), eine Kunststoffleiste (4), die ein erstes Längsende (8), das an dem optischen Körper (2) befestigt ist, und ein zweites Längsende (9) aufweist, an dem die Klammer (7) befestigt ist, und das eine Übergangstemperatur von mehr als 40°C aufweist, was einer Glasübergangstemperatur der Kunststoffleiste (4) oder einer Schmelztemperatur der Kunststoffleiste (4) entspricht, und das mindestens eine Verstrebung (5, 6), die ein erstes Längsende (10, 12) aufweist, das an dem optischen Körper (2) befestigt ist, und ein zweites Längsende (11, 13) aufweist, an dem die Klammer (7) befestigt ist, **dadurch gekennzeichnet, dass** die mindestens eine Verstrebung (5, 6) ein Bimetall umfasst.

2. Intraokularlinse nach Anspruch 1, wobei die Klammer (7) nur über die Kunststoffleiste (4) und die mindestens eine Verstrebung (5, 6) an dem optischen Körper (2) befestigt ist.

3. Intraokularlinse nach Anspruch 1 oder 2, wobei die mindestens eine Verstrebung (5, 6) in einem Abstand von der Kunststoffleiste (4) angeordnet ist.

4. Intraokularlinse nach einem der Ansprüche 1 bis 3, wobei das haptische Element (3) zwei der Verstrebungen (5, 6) aufweist, die in Umfangsrichtung auf beiden Seiten der Kunststoffleiste (4) in Bezug auf die optische Achse (19) des optischen Körpers (2) angeordnet sind.

5. Intraokularlinse nach einem der Ansprüche 1 bis 4, wobei die Intraokularlinse (1) zwei der haptischen Elemente (3) aufweist.

## Revendications

1. Lentille intraoculaire avec un corps optique (2) et un élément haptique (3) ayant une pince (7), une barre en plastique (4) qui a une première extrémité longitudinale (8) fixée au corps optique (2) et une seconde extrémité longitudinale (9) à laquelle la pince (7) est fixée et qui a une température de transition supérieure à 40 °C, soit une température de transition vitreuse de la barre en plastique (4) ou une température de fusion de la barre en plastique (4), et au moins une barre de renfort (5, 6) ayant une première extrémité longitudinale (10, 12) fixée au corps optique (2) et une seconde extrémité longitudinale (11, 13) à laquelle est fixée la pince (7), **caractérisée en ce que** l'au moins une barre de renfort (5, 6) comprend un bimétal.

2. Lentille intraoculaire selon la revendication 1, la pince (7) étant fixée au corps optique (2) uniquement par l'intermédiaire de la barre en plastique (4) et de l'au moins une barre de renfort (5, 6).

3. Lentille intraoculaire selon la revendication 1 ou 2, l'au moins une barre de renfort (5, 6) étant agencée à distance de la barre en plastique (4).

4. Lentille intraoculaire selon l'une quelconque des revendications 1 à 3, l'élément haptique (3) ayant deux des barres de renfort (5, 6) disposées dans la direction circonférentielle de chaque côté de la barre en plastique (4) par rapport à l'axe optique (19) du corps optique (2).

5. Lentille intraoculaire selon l'une quelconque des revendications 1 à 4, la lentille intraoculaire (1) ayant deux des éléments haptiques (3).
